# EUROPÄISCHE PATENTSCHRIFT

(11) **EP 0 721 334 B1**
(45) Veröffentlichungstag und Bekanntmachung des Hinweises auf die Patenterteilung: **07.01.1999**
(21) Anmeldenummer: 94928402.0
(22) Anmeldetag: 30.09.1994
(51) Int. Cl.: A61K 9/70, A61K 31/57, A61K 31/565

(54) **TRANSDERMALES THERAPEUTISCHES SYSTEM ENTHALTEND OCTYLDODECANOL ALS KRISTALLISATIONSINHIBITOR**
TRANSDERMAL THERAPEUTIC SYSTEM CONTAINING OCTYL DODECANOL AS CRYSTALLIZATION INHIBITOR
SYSTEME THERAPEUTIQUE TRANSDERMIQUE CONTENANT DE L'OCTYLDODECANOL COMME INHIBITEUR DE CRISTALLISATION

(30) Priorität: 01.10.1993 DE 4333595
(43) Veröffentlichungstag der Anmeldung: 17.07.1996
(73) Patentinhaber: Rotta Research B.V., 1017 PS Amsterdam (NL)
(72) Erfinder: CORDES, Günter, D-42799 Leichlingen (DE); ROVATI, Lucio, C., I-20022 Monza (MI) (IT)
(74) Vertreter: Boeters, Hans Dietrich, Dr.
(86) Internationale Anmeldenummer: EP9403269
(87) Internationale Veröffentlichungsnummer: WO9509618

(56) Entgegenhaltungen:
- EP-A- 0 013 949
- EP-A- 0 285 563
- EP-A- 0 416 842
- WO-A-93/08795
- FR-A- 2 612 785
- DATABASE WPI Section Ch, Week 8629, Derwent Publications Ltd., London, GB; Class B01, AN 86-186009 & JP,A,1 118 315 (HOKURIKU PHARM KK) 5. Juni 1986

## Beschreibung

Die vorliegende Erfindung betrifft ein transdermales therapeutisches System (TTS) zur Applikation von Arzneimitteln auf die Haut, und zwar von Estradiol und Norethisteronacetat.

Die TTS dienen unter anderem der Verabreichung bestimmter Hormone an den Menschen, um den im Laufe des Alterns zurückgehenden Hormonspiegel anzuheben. Im allgemeinen besteht ein derartiges Pflaster aus einer Trägerfolie, einem Haftkleber mit den Wirkstoffen und Hilfsstoffen, einem fakultativen weiteren Kleber im Form einer Schicht zur Erhöhung der Hafteigenschaften und schließlich einer Schutzfolie, die vor Gebrauch des Pflasters abgezogen und entfernt wird. Die Wirkstoffe gelangen durch die Haut in den Körper.

In vielen Fällen ist es bei der Verabreichung von Wirkstoffen aus TTS problematisch, einen ausreichend hohen Flux durch die Haut und damit einen genügend hohen Blutspiegel zu erzielen. Man begegnet diesem Problem entweder dadurch, daß man geeignete Enhancer hinzufügt, die die Durchlässigkeit der Haut für den Wirkstoff verbessern, oder man wählt eine möglichst hohe Wirkstoffkonzentration im Haftkleber bzw. in der den Wirkstoff enthaltenden Matrix. Durch die hohe Wirkstoffkonzentration soll eine hohe thermodynamische Aktivität erzielt und somit eine höhere Permeation durch die Haut ermöglicht werden. Durch diese Maßnahmen bewegt man sich an der Löslichkeits- bzw. Sättigungsgrenze des Wirkstoffs in der Matrix, die dabei überschritten werden kann. Da diese Grenze nicht immer exakt festzulegen oder einzuhalten ist und man sich bei dem Bemühen, die Wirkstoffkonzentration möglichst hoch zu wählen, im oder oberhalb des Grenzbereichs befinden kann, so kann es bei der Lagerung von TTS nach einigen Wochen oder Monaten zu Kristallisationserscheinungen kommen. Derartige Kristallisationserscheinungen sind ein für den Fachmann auf dem TTS-Gebiet gut bekanntes Phänomen.

Die DE-A-4 020 144 beschreibt beispielsweise ein TTS-System für verschiedene Wirkstoffe wie Estradiol und Norethisteronacetat (Seite 4, Zeilen 27 bis 28), wobei die selbstklebende Matrix-Schicht durch einen Polyacrylatkleber (Anspruch 1) vorgesehen wird. Als Enhancer bzw. Penetrationsbeschleuniger wird beispielsweise n-Dodecanol vorgeschlagen (Seite 5, Zeile 53). Das Kristallisationsproblem wird nicht angesprochen. Die DE-A-3 933 460 betrifft ein TTS für Hormone, wie Estradiol und Norethisteron (Seite 4, Zeilen 26 bis 51), wobei als Haftkleber Homo- und/oder Copolymere mit mindestens einem Derivat der Acryl- oder Methacrylsäure vorgesehen werden (Seite 3 Abs. 3 und ff). Eine zweckmäßige Ausführungsform kann Stoffe enthalten, die die Kristallisation des Wirkstoffs verzögern oder verhindern und die in einer Konzentration von 0,1 bis 20 Gew.-% enthalten sind, wobei als Kristallisationsverzögerer Phthalsäureester, Adipinsäureester, Mono-, Di- und Triglyceride, Ester höherer Fettsäuren, langkettige Alkohole und deren Derivate, Derivate des Nonylphenols bzw. des Octylphenols, Derivate von Fettsäuren, Derivate des Sorbits und des Mannits, nichtionische Tenside, Polyoxyethylenalkylether, Derivate des Rizinusöls, Sitosterin und Polyvinylpyrrolidon angesprochen werden (Seite 3 Abs. 2 und Seite 4 Abs. 2). Die DE-A-3 810 896 schlägt ein TTS vor, bei dem beispielsweise Estradiol and Norethisteronacetat in einem Reservoir vorgesehen werden. Auch Penetrationsverbesserer werden genannt (Seite 3, Zeile 39 und Seite 5, Zeile 50 und ff). Kristallisationsprobleme werden nicht angesprochen. Die US-A-5 198 223 betrifft ein transdermales therapeutisches System für beispielsweise Estradiol (Spalte 7, vorletzter Absatz), bei dem auch Penetrationsenhancer vorgesehen werden können (Spalte 6, Abs. 4 und ff). Kristallisationsprobleme werden nicht angesprochen. Mit der EP-A-0 416 842 wird ein transdermales therapeutisches Matrix-System für beispielsweise Estradiol (Seite 4, Zeile 2) vorgesehen, wobei betont wird, daß man von Penetrationsenhancern absehen kann, wenn man die vorgeschlagene Matrix einsetzt (Seite 3, Zeilen 33 bis 37). Kristallisationsprobleme werden nicht angesprochen. Die WO-A-93/10 772 beschreibt ein estradiolhaltiges transdermales therapeutisches System, für dessen Haftkleber Acrylatcopolymere vorgeschlagen werden (Ansprüche 1 und 2). Kristallisationsinhibitoren werden nicht angesprochen. Das bekannte System will jedoch durch seine spezielle Ausbildung eine Kristallisation vermeiden.

Aufgabe der vorliegenden Erfindung ist es, ein transdermales therapeutisches System zur Applikation von Estradiol und Norethisteronacetat vorzusehen, mit dem bei hoher Wirkstoffkonzentration Kristallisationserscheinungen noch befriedigender vermieden werden können.

Die der Erfindung zugrundeliegende Aufgabe wird nun durch ein transdermales therapeutisches System gelöst, das
- eine Trägerfolie
- einen Haftkleber auf Acrylatbasis als Matrix, in der
- als Wirkstoffe Estradiol und Norethisteronacetat (NETA) gelöst sowie
- Octyldodecanol vorliegen,
- gegebenenfalls eine weitere Schicht eines Haftklebers sowie
- eine Schutzfolie
umfaßt oder aus diesen Komponenten besteht.

Die vorliegende Erfindung sieht also den Zusatz eines bestimmten Hilfsstoffs, nämlich Octyldodecanol, zur Polymer-Klebemasse mit den darin gelösten Wirkstoffen vor, um auf diese Weise Kristallisationserscheinungen der Wirkstoffe auch nach Lagerung herabzusetzen. Damit kann eine hohe thermodynamische Aktivität aufrechterhalten werden, ohne das Risiko einer Kristallbildung in Kauf nehmen zu müssen. Octyldodecanol ist im Handel als Eutanol G erhältlich.

Die Trägerfolie des erfindungsgemäßen TTS kann aus Polyethylenterephthalat bestehen.

Der erfindungsgemäß einzusetzende Haftkleber auf Acrylatbasis kann durch radikalische Mischpolymerisation von
2-Ethylhexylacrylat und/oder
Methylacrylat und/oder
Acrylsäure und/oder
Vinylacetat und/oder Hydroxyethylacrylat und
gegebenenfalls bis zu 2 % an anderen Substanzen hergestellt worden sein.

Beispielsweise kann der Haftkleber auf Acrylatbasis durch radikalische Mischpolymerisation von
2-Ethylhexylacrylat in einer Menge von 50 bis 70 und insbesondere 55 bis 65 %,
Methylacrylat in einer Menge von 24 bis 32 %,
Acrylsäure in einer Menge von 2 bis 8 %,
Vinylacetat in einer Menge von 2 bis 10 % und
Hydroxyethylacrylat in einer Menge von 0,5 bis 3 % hergestellt worden sein (jeweils bezogen auf das Matrixgewicht).

Für den erfindungsgemäß verwendbaren Haftkleber auf Acrylatbasis kann auch voll inhaltlich auf WO-A-93/10 772 verwiesen werden.

Gemäß einer bevorzugten Ausführungsform kann es sich bei dem erfindungsgemäß verwendbaren Haftkleber auf Acrylatbasis um ein Gemisch von zwei oder mehr Haftklebern handeln, wie sie vorstehend beschrieben worden sind.

Gemäß einer bevorzugten Ausführungsform kann ein cm² des erfindungsgemäßen transdermalen therapeutischen Systems folgende Komponenten enthalten:
0,05 bis 0,5 mg Estradiol,
0,5 bis 1,5 mg Norethisteronacetat,
0,1 bis 0,5 mg Octyldodecanol und
5 bis 12 mg eines Haftklebers auf Acrylatbasis.

Die Schutzfolie des erfindungsgemäßen TTS kann aus Polyethylenterephthalat bestehen.

Es sei nochmals hervorgehoben, daß bei dem erfindungsgemäßen transdermalen therapeutischen System die Wirkstoffe gelöst und nicht kristallin vorliegen.

### Beispiel 1

Es werden 14 g Estradiol und 92 g Norethisteronacetat unter Rühren in 1200 g Ethylmethylketon gelöst. Unter fortgesetztem Rühren werden 23,7 g Octyldodecanol hinzugefügt. Danach gibt man 360 g einer 51-proz. Lösung (G/V) eines ersten Acrylat-Copolymeren (Durotac 280-2287 von National Starch Chemical B.V. (Zutphen/Niederlande) und 2000 g einer 37-proz. Lösung eines weiteren Acrylat-Copolymeren (Durotac 326-1753 von National Starch Chemical B.V.) hinzu und löst unter Rühren. Nachdem eine homogene Lösung entstanden ist, streicht man sie auf eine silikonisierte Polyesterfolie (100 µm) aus. Danach läßt man das Lösungsmittel verdunsten, wobei man gegebenenfalls auf etwa 40 °C erwärmt, und deckt auf der Kleberseite mit einer Polyesterfolie (15 µm) ab. Die einzelnen TTS werden in der gewünschten Größe in üblicher Weise ausgestanzt, beispielsweise in einer Größe von 20 bis 50 cm².

### Beispiel 2 und Vergleichsbeispiel 1

Durch eine Lagerung bei 40 °C und 75 % relativer Feuchtigkeit kann man die Kristallisation der beiden Wirkstoffe stark beschleunigen. Durch eine derartige extreme Behandlung der Polymermatrix läßt sich nach relativ kurzer Zeit zeigen, daß ein erfindungsgemäßes TTS mit einem Gehalt Octyldodecanol einem TTS ohne Octyldodecanol überlegen ist. Für diesen Vergleich wurde Beispiel 1 folgendermaßen wiederholt.

### Vergleichsbeispiel 1:

- 5,8 mg: Estradiol
- 35,8 mg: Norethisteronacetat
- 362,0 mg: Polyacrylat-Gemisch

### Beispiel 2:

- 5,8 mg: Estradiol
- 35,8 mg: Norethisteronacetat
- 362,0 mg: Polyacrylat-Gemisch
- 9,3 mg: Octyldodecanol

**Tabelle 1**

| | Vergleichs-beispiel 1 | Beispiel 2 |
|---|---|---|
| Lagerung bei 40 °C/75 % RF | 2 Wochen | 2 Wochen |
| Anzahl der Kristalle/cm² | 27 | 5 |
| Kristallgröße | 0,4 mm | 0,25 mm |

Ein Vergleich der Beispiele belegt, daß Octyldodecanol Größe und Anzahl der Kristalle in der Klebstoffmatrix unter den Versuchsbedingungen deutlich verringert. Bei Lagerung bei Raumtemperatur bilden sich Kristalle erst nach weitaus längerer Zeit, wobei sie bei Beispiel 2 ganz ausbleiben würden.

## Patentansprüche

1. Transdermales therapeutisches System, umfassend oder bestehend aus
- einer Trägerfolie,
- einem Haftkleber auf Acrylatbasis als Matrix, in der
- als Wirkstoffe Estradiol und Norethisteronacetat gelöst sowie
- Octyldodecanol vorliegen,
- gegebenenfalls einer weiteren Schicht eines Haftklebers sowie
- einer Schutzfolie.

2. Transdermales therapeutisches System nach Anspruch 1, dadurch **gekennzeichnet**, daß die Trägerfolie aus Polyethylenterephthalat besteht.

3. Transdermales therapeutisches System nach Anspruch 1 oder 2, **dadurch gekennzeichnet**, daß die Wirkstoffe gelöst und unkristallisiert vorliegen.

4. Transdermales therapeutisches System nach einem der vorhergehenden Ansprüche, **gekennzeichnet** durch einen Haftkleber auf Acrylatbasis, der durch radikalische Mischpolymerisation von
2-Ethylhexylacrylat und/oder
Methylacrylat und/oder
Acrylsäure und/oder
Vinylacetat und/oder
Hydroxyethylacrylat und
gegebenenfalls bis zu 2 % an anderen Substanzen hergestellt worden ist.

5. Transdermales therapeutisches System nach Anspruch 4, **gekennzeichnet** durch einen Haftkleber auf Acrylatbasis, der durch radikalische Mischpolymerisation von
2-Ethylhexylacrylat in einer Menge von 50 bis 70 % und insbesondere 55 bis 65 %,
Methylacrylat in einer Menge von 20 bis 40 % und insbesondere 24 bis 32 %,
Acrylsäure in einer Menge von 2 bis 8 %,
Vinylacetat in einer Menge von 2 bis 10 %,
Hydroxyethylacrylat in einer Menge von 0,5 bis 3 % und
gegebenenfalls bis zu 2 % an anderen Substanzen hergestellt worden ist (jeweils bezogen auf das Matrixgewicht).

6. Transdermales therapeutisches System nach einem der vorhergehenden Ansprüche, dadurch **gekennzeichnet**, daß es sich bei dem Haftkleber auf Acrylatbasis um ein Gemisch von zwei oder mehr Haftklebern gemäß Anspruch 4 oder 5 handelt.

7. Transdermales therapeutisches System gemäß einem der vorhergehenden Ansprüche, dadurch **gekennzeichnet**, daß 1 cm² des Systems folgende Komponenten enthält:
0,1 bis 0,5 mg Octyldodecanol,
0,05 bis 0,5 mg Estradiol,
0,5 bis 1,5 mg Norethisteronacetat und
5 bis 12 mg eines Haftklebers auf Acrylatbasis.

## Claims

1. Transdermal therapeutic system comprising or consisting of
- a carrier film
- a pressure sensitive adhesive based on acrylate as matrix in which
- estradiol and norethisterone acetate (NETA) as active substances in the dissolved state and
- octyl dodecanol are present
- if necessary, a further layer of a pressure sensitive adhesive and
- a protective film

2. Transdermal therapeutic system according to claim 1 characterised in that the carrier film consists of polyethylene terephthalate.

3. Transdermal therapeutic system according to claim 1 or 2 characterised in that the active substances are present in the dissolved and non-crystallised state.

4. Transdermal therapeutic system according to one of the preceding claims characterised by a pressure sensitive adhesive based on acrylate which has been produced by the radical copolymerisation of
2-ethyl hexyl acrylate and/or
methyl acrylate and/or
acrylic acid and/or
vinyl acetate and/or
hydroxyethyl acrylate and
if necessary, up to 2% of other substances.

5. Transdermal therapeutic system according to claim 4 characterised by a pressure sensitive adhesive based on acrylate which has been produced by the radical copolymerisation of
2-ethyl hexyl acrylate in a quantity of 50 to 70% and in particular 55 to 65%,
methyl acrylate in a quantity of 20 to 40% and in particular of 24 to 32%,
acrylic acid in a quantity of 2 to 8%,
vinyl acetate in a quantity of 2 to 10%,
hydroxyethyl acrylate in a quantity of 0.5 to 3% and
if necessary, up to 2% of other substances (based on the weight of the matrix in each case).

6. Transdermal therapeutic system according to one of the preceding claims characterised in that the pressure sensitive adhesive based on acrylate is a mixture of two or more pressure sensitive adhesives according to claim 4 or 5.

7. Transdermal therapeutic system according to one of the preceding claims characterised in that 1 cm² of the system contains the following components:
0.1 to 0.5 mg octyl dodecanol,
0.05 to 0.5 mg estradiol,
0.5 to 1.5 mg norethisterone acetate and
5 to 12 mg of a pressure sensitive adhesive based on acrylate.

## Revendications

1. Système thérapeutique transdermique constitué de ou comprenant
- une feuille de support,
- une matrice à base d'un adhésif acrylique, dans laquelle sont dissous
- les principes actifs estradiol et acétate de noréthistérone (NETA), et dans laquelle est présent
- de l'octyldodécanol,
- éventuellement une couche supplémentaire d'un adhésif, ainsi que
- une feuille protectrice.

2. Système thérapeutique transdermique selon la revendication 1, caractérisé en ce que la feuille de support est constituée de poly(éthylène téréphtalate).

3. Système thérapeutique transdermique selon la revendication 1 ou 2, caractérisé en ce que les principes actifs sont présents sous forme dissoute et non cristallisée.

4. Système thérapeutique transdermique selon l'une quelconque des revendications précédentes, caractérisé en ce que l'adhésif acrylique est préparé par copolymérisation radicalaire
d'acrylate de 2-éthylhexyle et/ou
d'acrylate de méthyle et/ou
d'acide acrylique et/ou
d'acétate de vinyle et/ou
d'acrylate d'hydroxyéthyle,
et éventuellement, de jusqu'à 2 % d'autres substances.

5. Système thérapeutique transdermique selon la revendication 4, caractérisé en ce que l'adhésif acrylique est préparé par copolymérisation radicalaire
- de 50 à 70 %, en particulier de 55 à 65 %, d'acrylate de 2-éthylhexyle,
- de 20 à 40 %, en particulier de 24 à 32 %, d'acrylate de méthyle,
- de 2 à 8 % d'acide acrylique,
- de 2 à 10 % d'acétate de vinyle, et
- de 0,5 à 3 % d'acrylate d'hydroxyéthyle,
et éventuellement, de jusqu'à 2 % d'autres substances,
tous ces pourcentages étant rapportés au poids total de la matrice.

6. Système thérapeutique transdermique selon l'une des revendications précédentes, caractérisé en ce que l'adhésif acrylique est un mélange de deux ou plusieurs adhésifs selon les revendications 4 ou 5.

7. Sytème thérapeutique transdermique selon l'une quelconque des revendications précédentes, caractérisé en ce que 1 cm² du système contient les composants suivants :
de 0,05 à 0,5 mg d'estradiol,
de 0,5 à 1,5 mg d'acétate de noréthistérone,
de 0,1 à 0,5 mg d'octyldécanol et
de 5 à 12 mg d'un adhésif acrylique.
